# EUROPEAN PATENT APPLICATION

(11) **EP 4 501 215 A1**
(43) Date of publication of application: **05.02.2025**
(21) Application number: 24191314.4
(22) Date of filing: 26.07.2024
(51) Int. Cl.: A61B 5/00, A61B 5/11

(54) **SYSTEMS AND METHODS OF BIOMECHANICAL EVALUATION OF ATHLETIC PERFORMANCE**

(30) Priority: 03.08.2023 US 202363517541 P; 19.07.2024 US 202418777671
(71) Applicant: Amtote International, Inc., Hunt Valley, MD 21031 (US)
(72) Inventor: BIEMER, Garrett, Boston, 02132 (US); WILLIAMS, Paul, Boca Raton, 33496 (US)
(74) Representative: Openshaw & Co.

(57) **Abstract**

A method for assessing the biomechanics of an animal including applying, to a biomechanic evaluation model, i) a tracked position of biomechanic markers, and ii) a determined biomechanic metric, to generate an output including at least one biomechanic assessment metric for use in assessing the performance of the identified animal.

## Description

### RELATED APPLICATIONS

This application claims priority to U.S. Provisional Application Serial No. 63/517,541, filed on August 3, 2023, and U.S. Non-Provisional Application Serial No. 18/777671, filed on July 19, 2024, the contents of which are hereby incorporated by reference in their entirety.

### BACKGROUND

The field of the disclosure relates generally to biomechanical evaluation of athletic performance and more particularly, to methods and systems for evaluating the biomechanical performance of an animal to assess pathology or injury.

With the recent advances in technology, it has become more common for athletes to be biometrically evaluated and monitored by trained medical personnel. Such evaluations enable an understanding of the relationship between body parts and tissues that may be moving too much or which may be not moving enough, allow an understanding of what is tight on the athlete and what is loose, and allow an understanding of what is weak and what is strong on the athlete, for example. Moreover, completing such evaluations enables a training regime and an action to be developed, and can play a crucial role in both injury prevention as well as performance enhancement.

Similarly, horses, and more particularly racehorses, may be monitored, either by an animal trainer or a veterinarian, to ensure that the horse has optimal biomechanical performance, to diagnose injury or pathology, and/or to evaluate the efficacy of treatments or intervention protocols. However, the effectiveness of performing biomechanical evaluations with racehorses may be limited. For example, in some cases, dozens of racehorses may exercise, train, and/or race about a track, at the same time such that an unobstructed view of the track at all regions of the track may be difficult for a person monitoring a specific racehorse. Furthermore, monitoring a racehorse may be time consuming, and trainers or veterinarians may not have the capacity to monitor an animal each time the animal is training, exercising, or racing. Furthermore, even an experienced trainer or veterinarian may not have the ability to visually detect pathology or injury during the early stages of pathology or disease.

Accordingly, a need exists for systems and methods that may be used to evaluate the biomechanical performance of an animal, without the aforementioned drawbacks of conventional monitoring methods.

### SUMMARY

In one aspect, a computer system for assessing the biomechanical performance of an animal is provided. The computer system includes at least one processor in communication with at least one memory device. The processor is programmed to receive current image data from at least one camera of at least one animal and identify with specificity an animal within the received image data. The processor is further configured to identify at least one biomechanic marker on the identified animal and track the position of each identified biomechanic marker. The processor further configured to determine a biomechanic metric based on each identified biomechanic marker and apply, to a biomechanic evaluation model, i) the tracked position of each biomechanic marker, and ii) the determined biomechanic metric, to generate an output including at least one biomechanic assessment metric for use in assessing the performance of the identified animal. The processor further configured to transmit one or more notification messages to at least one computing device, wherein each notification message includes at least one generated output biomechanic assessment metric.

In another aspect, a method for assessing the biomechanics of an animal is provided. The method implemented using a computer device including a processor in communication with a memory device. The method includes receiving current image data from at least one camera of at least one animal and identifying with specificity an animal within the received image data. The method includes identifying at least one biomechanic marker on the identified animal and tracking the position of each identified biomechanic marker. The method includes determining a biomechanic metric based on each identified biomechanic marker and applying, to a biomechanic evaluation model, i) the tracked position of each biomechanic marker, and ii) the determined biomechanic metric, to generate an output including at least one biomechanic assessment metric for use in assessing the performance of the identified animal. The method includes transmitting one or more notification messages to at least one computing device, wherein each notification message includes at least one generated output biomechanic assessment metric.

In another aspect, a non-transitory computer-readable storage medium including computer-executable instructions embodied thereon for assessing the biomechanics of an animal is provided. The computer executable instructions cause the at least one processor to receive current image data from at least one camera of at least one animal and identify with specificity an animal within the received image data. The computer executable instructions cause the at least one processor to identify at least one biomechanic marker on the identified animal and track the position of each identified biomechanic marker. The computer executable instructions cause the at least one processor to determine a biomechanic metric based on each identified biomechanic marker and apply, to a biomechanic evaluation model, i) the tracked position of each biomechanic marker, and ii) the determined biomechanic metric, to generate an output including at least one biomechanic assessment metric for use in assessing the performance of the identified animal. The computer executable instructions cause the at least one processor to transmit one or more notification messages to at least one computing device, wherein each notification message includes at least one generated output biomechanic assessment metric.

### BRIEF DESCRIPTION OF THE DRAWINGS

These and other features, aspects, and advantages of the present disclosure will become better understood when the following detailed description is read with reference to the accompanying drawings in which like characters represent like parts throughout the drawings, wherein:
FIG. 1 is a schematic diagram of an exemplary biomechanical evaluation system for use in assessing the biomechanical performance of an animal.
FIG. 2 is a schematic illustration of an exemplary track and camera system that may be used with the biomechanical evaluation system shown in FIG. 1.
FIG. 3 is a first view of a graphical user interface (GUI) that may be used with the biomechanical evaluation system shown in FIG. 1.
FIG. 4 is a second view of a graphical user interface (GUI) that may be used with the biomechanical evaluation system shown in FIG. 1.
FIG. 5 is a flow chart illustrating an exemplary process for assessing the biomechanical performance of an animal, which may be implemented using the system shown in FIG. 1.
FIG. 6 is a flow diagram illustrating an exemplary method that may be implemented to assess the biomechanical performance of an animal, using the system shown in FIG. 1.
FIG. 7 is a graphic illustrating exemplary average absolute head amplitude (normalized) versus standard deviation of absolute head amplitude (normalized) and standard deviation of frequency.
FIG. 8 illustrates an exemplary configuration of a user system, such as the veterinarian computing device shown in FIG. 1.
FIG. 9 illustrates an exemplary configuration of a server system, such as the system computing device shown in FIG. 1.

Unless otherwise indicated, the drawings provided herein are meant to illustrate features of embodiments of the disclosure. These features are believed to be applicable in a wide variety of systems comprising one or more embodiments of the disclosure. As such, the drawings are not meant to include all conventional features known by those of ordinary skill in the art to be required for the practice of the embodiments disclosed herein.

### DETAILED DESCRIPTION

In the following specification and the claims, reference will be made to a number of terms, which shall be defined to have the following meanings. The singular forms "a," "an," and "the" include plural references unless the context clearly dictates otherwise. "Optional" or "optionally" means that the subsequently described event or circumstance may or may not occur, and that the description includes instances where the event occurs and instances where it does not. Furthermore, references to "one embodiment" are not intended to be interpreted as excluding the existence of additional embodiments that also incorporate the recited features. Moreover, unless explicitly stated to the contrary, embodiments "including" or "having" an element or a plurality of elements having a particular property may include additional such elements not having that property.

As used herein, the term "real-time" refers to either the time of occurrence of the associated events, the time of measurement and collection of predetermined data, the time to process the data, or the time of a system response to the events and the environment. In the embodiments described herein, these activities and events occur substantially instantaneously.

Embodiments described herein include a biomechanical evaluation system for evaluating the biomechanical performance of an animal, such as a horse. In the exemplary embodiments, the biomechanical evaluation system includes at least one camera system for collecting image data of the animal as it moves about a racetrack, e.g., during a race, during training, and/or during exercising. Each camera system includes a plurality of cameras positioned about the track in order to capture image data of animals as the animals move around the track. In some embodiments described herein, the biomechanical evaluation system also includes an identification system that automatically identifies a specific animal captured in the image data, even if a plurality of animals is contained in the image data. Moreover, in some embodiments described herein, the system automatically identifies anatomical markers on the animals captured in the image data such that the relative position and/or displacement of the anatomical markers can be tracked as the animal moves about the track. In some embodiments described herein, the system may use the tracked position of the anatomical markers to determine one or more biomechanic metrics such as speed, acceleration, stride length, and/or gait type.

In some embodiments described herein, the system generates a biomechanical evaluation model that accepts one or more inputs in order to generate one or more outputs including a biomechanical assessment metric. The generated biomechanical assessment metric may be used to evaluate the biomechanical performance of the animal, to assess pathologies or injuries of the animal, and/or to predict potential injuries of the animal. The biomechanical evaluation system may collect image data of the animal during an elapsed period of time, e.g., during weeks or months, to determine a normal or baseline biomechanical performance of the animal, *e.g.,* a top speed and/or a gait pattern unique to a specific animal. As such, the biomechanical evaluation model may be used to detect changes in the animal's normal gait pattern.

The biomechanical evaluation models may be used to evaluate the health or biomechanical performance of the animal, such as, and without limitation, injury, pathology, any change in the animal's normal gait, or changes in the animal's behavior or movement patterns, *e.g*., changes in head movement.

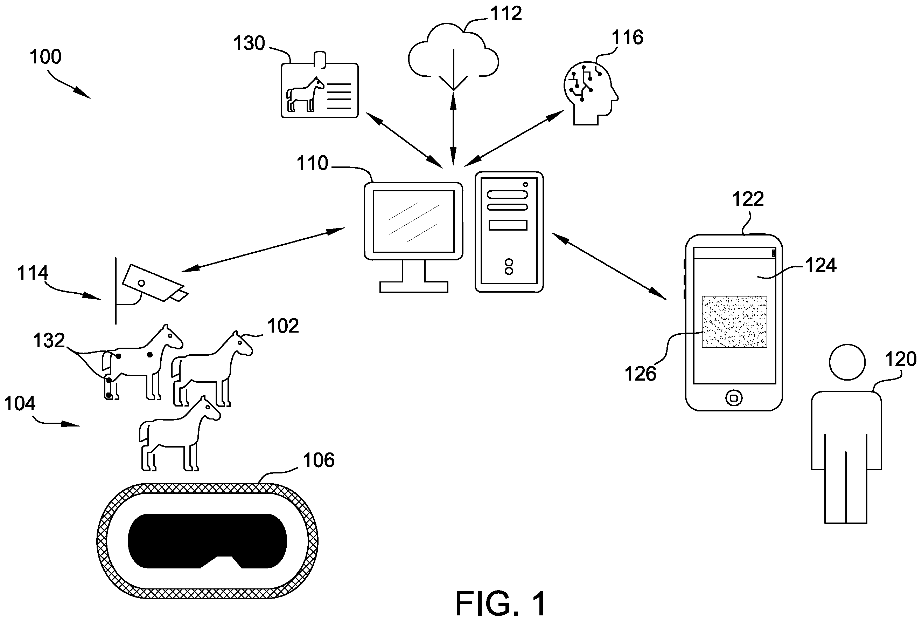

Referring now to the drawings, FIG. 1 is a schematic illustration of an exemplary biomechanical evaluation (BE) system 100 that may be used to evaluate the biomechanics of an animal 102 or of a plurality of animals 104. In the exemplary embodiment, the BE system 100 is integrated within a track 106 in which at least one the animals 104 moves around the track 106, *e.g*., exercises, trains, walks, and/or competes on the track 106. In other embodiments, the BE system 100 may be integrated within another type of enclosure or location, such as a pasture wherein the animals 104 may move within the contained area.

In the exemplary embodiment, the BE system 100 includes a system computing device 110 that may be communicatively coupled to a storage database 112. In some embodiments, the storage database 112 may be a cloud-based database 112. In the embodiments described herein, the BE system 100 includes a camera system 114 used to collect image data, *e.g.,* image frames and/or a series of image frames such as a video feed, of the animals 104 as they move about the track 106. In the embodiments described herein, the BE system 100 includes a biomechanical evaluation (BE) model 116 that is continuously trained and that may be used to determine one or more biomechanical assessment metrics associated with a subject being monitored. For example, the assessment metrics may be used to evaluate the biomechanical performance of an animal 102, diagnose injuries and/or pathologies, predict possible injuries of an animal, track progression of injuries and/or pathologies, and/or to evaluate the effectiveness of treatment, training, and/or intervention strategies. The BE model 116 and the assessment metrics are described in more detail herein.

In the exemplary embodiments, the animal 102 may be a horse, such as a racing horse. In other embodiments, the BE system 100 may be used to evaluate the biomechanics of other types of animals 104 such as, but not limited to, a dog, a camel, an elephant, and/or any other animal that may be auctioned and/or raced, for example. In addition, in other embodiments, a veterinarian and/or a zookeeper may use BE system 100 to evaluate animals kept in captivity, and/or that are difficult or risky to otherwise approach, for example.

In some embodiments, the system computing device 110 may be communicatively coupled to one or more other computing devices, associated with one or more interested parties or person(s) 120, such as, for example and without limitation, a veterinarian, an animal trainer, and/or any other party such, but not limited to, the animal's owner and/or a rider, i.e., a jockey, for example, of the animal. The interested parties or person(s) 120 may refer to any party interested in receiving information regarding the biomechanical performance of an animal. In some embodiments, the interested parties may be required to complete an enrollment process prior to being authorized to participate with the BE system 100. The interested parties may be associated with a computing device, referred to herein as a veterinarian computing device 122. The system computing device 110 and/or the veterinarian computing device 122 may include a user interface 124, *e.g.,* a visual and/or audio display, enabling a graphical user interface (GUI) 126 to present information to the interested parties 120, referred to herein as the veterinarian 120. The system computing device 110 may be in communication with other computing devices through an application program interface (API) and/or through an associated, *e.g.,* a front end, GUI 126, which enables the exchange of data and/or messages. For example, the system computing device 110 may transmit one or more messages, including video feed and/or assessment metrics, to the other computing devices and/or may cause the other computing devices to display one or more views of the GUI 126.

The computing devices, *e.g.,* the system computing device 110 and the veterinarian computing device 122, may be communicatively coupled to the Internet through a plurality of different interfaces including, but not limited to, at least one of a network, such as the Intranet, a local area network (LAN), a wide area network (WAN), or an integrated services digital network (ISDN), a dial-up-connection, a digital subscriber line (DSL), a cellular phone connection, and/or a cable modem. The system computing device 110 and/or the veterinarian computing device 122 may include any device capable of accessing the Internet including, but is not limited to, a desktop computer, a laptop computer, a personal digital assistant (PDA), a cellular phone, a smartphone, a tablet, a phablet, and/or other web-based connectable equipment.

As used herein, the term "database" may refer to either a body of data, a relational database management system (RDBMS), or to both. A database may include any collection of data including hierarchical databases, relational databases, flat file databases, object-relational databases, object-oriented databases, and any other structured collection of records or data that is stored in a computer system. The above examples are for example only, and thus are not intended to limit in any way the definition and/or meaning of the term database. Examples of RDBMS's include, but are not limited to including, Oracle^{®} Database, MySQL, IBM^{®} DB2, Microsoft^{®} SQL Server, Sybase^{®}, and PostgreSQL. However, any database may be used that enables the systems and methods described herein. (Oracle is a registered trademark of Oracle Corporation, Redwood Shores, California; IBM is a registered trademark of International Business Machines Corporation, Armonk, New York; Microsoft is a registered trademark of Microsoft Corporation, Redmond, Washington; and Sybase is a registered trademark of Sybase, Dublin, California).

The term processor, as used herein, may refer to central processing units, microprocessors, microcontrollers, reduced instruction set circuits (RISC), application specific integrated circuits (ASIC), logic circuits, and any other circuit or processor capable of executing the functions described herein.

As used herein, the terms "software" and "firmware" are interchangeable and include any computer program stored in memory for execution by a processor, including RAM memory, ROM memory, EPROM memory, EEPROM memory, and non-volatile RAM (NVRAM) memory. The above memory types are for example only and are thus not limiting as to the types of memory usable for storage of a computer program.

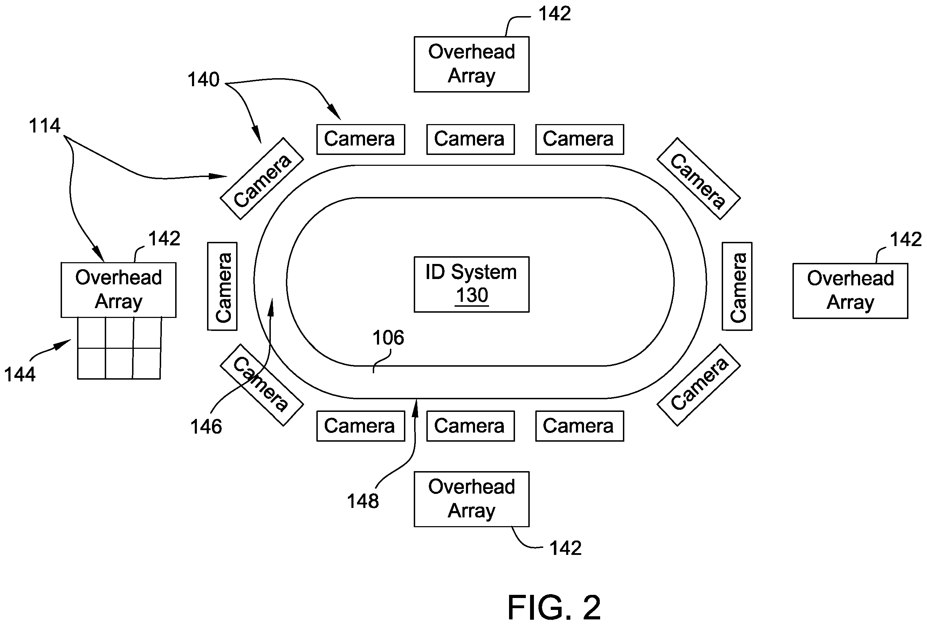

In the embodiments described herein, the BE system 100 may also include, or may be communicatively coupled to, an identification system 130 enabled to identify a specific animal, within the image data, even if a plurality of animals 104 are captured in the image data. In some embodiments, the identification system 130 is enabled to identify a specific animal 102 within image data, even if there is greater than 25 different animals, greater than 50 different animals, or greater than 100 different animals 104 captured in the image data, for example. The identification system 130 may be associated with one or more identification computing devices (not shown), that are independent from the system computing device 110, or alternatively, the identification system 130 may be incorporated within the system computing device 110. In the exemplary embodiment, the identification system 130 is also enabled to identify a specific animal 102, even as the animal 102 is moving between the field of view of adjacent cameras 140, 142, used in the camera system 114, described in detail with respect to FIG. 2. In some embodiments, the identification system 130 may use an identification marker (not shown) coupled to the animal such as, but not limited to a saddle, a saddle cloth or blanket, and/or a leg wrap. In some embodiments, the identification system 130 may include an identification (ID) model that is continuously trained to identify a specific animal 102 within collected image data. In some embodiments, the identification system 130 may use anatomical markers 132, described below, to determine one or more biometric body measurements for identifying a specific animal 102. The identification system 130 may utilize any suitable processes or techniques in order to identify a specific animal 102 in the image data. In some embodiments, the ID model may be run on a computing device separate from the system computing device 110, and after the ID model has identified an animal 102, then the model result and/or the image data containing the identified animal is transmitted to the system computing device 110. Accordingly, in some embodiments, not all collected image date is transmitted to the system computing device 110, rather only image data containing identified animals are transmitted to the system 100 for evaluation, thus reducing computational times and improving computational efficiency.

In the exemplary embodiments, the system computing device 110 may identify one or more anatomical markers 132 using the image data of an identified animal 102. In the exemplary embodiment, anatomical markers 132 may refer to one or more locations of particular interest that may be tracked (*e.g*., a position, a displacement, and/or rotations over time) using the camera system 114 and/or the system computing device 110. In the exemplary embodiments, the anatomical markers 132 may include, but are not limited to only including, joint centers, limb segments, centers of mass, and/or axes of rotation. In the exemplary embodiment, at least one anatomical marker 132 may be placed on, for example, a leg and/or foot of the horse, *e.g.,* a hoof, a heel, an ergot, and/or a fetlock, etc. In the exemplary embodiment, at least one anatomical marker 132 may be placed on the knee, the hip, and/or the elbow, for example. In some embodiments, the anatomical marker may be placed on the shoulder, on the crest, on the withers, and/or the head of the animal, for example. The BE system 100 may identify any other suitable anatomical marker132 that enables the BE system 100 to function as described herein.

The system computing device 110 may identify the anatomical markers 132, in each sequential image frame, in order to better track the relative position, and therefore, the motion, of the anatomical markers 132. In some embodiments, the system computing device 110 may track the relative position and/or motion of the anatomical markers 132 using a global coordinate system positioned on the track 106. In some embodiments, the system computing device 110 may track the relative position and/or the motion of the anatomical marker 132 in relation to a local coordinate system positioned on the body of the animal. In some alternative embodiments, the system computing device 110 may track the relative position and/or motion of the anatomical marker 132 in relation to one or more other anatomical markers 132, also referred to herein as relative anatomical marker motion. In some alternative embodiments, anatomical markers 132 may be positioned on a person, such as for example, a person that is riding the horse and/or on a saddle mounted to the animal. In some embodiments, a graphical location system (GPS) may be utilized to determine a location of an animal 102 and identify an animal 102 within image data. For example, GPS data may be obtained using Ultra-Wide-Band tag worn by the jockey and/or the animal or Phone App providing GPS data on the position of the animal 102.

In the exemplary embodiment, the system computing device 110 may determine one or more biomechanic metrics based at least in part, on the tracked position or movement of one or more of the anatomical markers 132. In the exemplary embodiment, the determined biomechanic metrics may include, without limitation, a velocity, an acceleration, and/or a jerk (i.e., the rate of change of acceleration) of one or more individual anatomical markers 132. In some embodiments, the determined biomechanic metric may include an average velocity, an acceleration, and/or a jerk of two or more anatomical markers 132. In some embodiments, determined metrics may include vertical displacement of one or more of the anatomical markers 132, *e.g.,* vertical displacement or range of displacement, of at least one anatomical marker 132 coupled to the head of the animal, for example.

In some embodiments, the determined biomechanic metrics may also include a stride length and/or a type of gait, such as, for example and without limitation, a walk, a trot, a canter, a gallop, a back, and/or a hand gallop. In some embodiments, the system computing device 110 is enabled to determine a type of gait based on a tracked position of at least one anatomical marker 132. In some embodiments, the system computing device 110 may determine the type of gait based on the motion of at least one anatomical markers 132 coupled to a foot or a leg of the animal 102 being evaluated, by determining when each of the hoofs, for example, (*e.g.*, left, or right front hoofs and/or left or right hind hoofs) are off the ground and/or contacting the ground. For example, the system computing device 110 may determine that the hoof anatomical markers 132 indicate that, in sequential order, the animal's right hind hood initially strikes the ground followed by their right front hoof, their left hind hoof, and their left front foot hoof strike the ground, wherein each hoof hits the ground independently, and therefore, the system computing device 110 recognizes this pattern as a walk gait associated with a specific animal. The system computing device 110 may be enabled to determine any suitable type of gait, using the tracked anatomical marker 132 and/or using any identifiable pattern of hoof motion. In some alternative embodiments, the type of gait may be determined using the BE model 116.

FIG. 2 is a schematic illustration of the camera system 114 and the track 106 used with the BE system 100 (shown in FIG. 1). The camera system 114 may include any device enabled to capture images, for example, and without limitation, a camera capable of capturing a plurality of image frames, a camera capable of capturing sequential image frames, a camera capable of capturing a video stream, and/or the like. The camera system 114 may include, but is not limited to only including, a neuromorphic camera, a digital camera, an infrared camera, a drone camera, a mirrorless camera, and or any other camera that enables BE system 100 to function as described herein. In the exemplary embodiment, the camera system 114 includes a plurality of trackside cameras 140 and a plurality of overhead cameras 142. Alternatively, system 114 may include any other camera locations that enables BE system 100 to function as described herein. For example, in some embodiments, system 114 may include point-of-view cameras worn by the rider of the animal being evaluated. The cameras 140 and/or 142 may collect image data, continuously and/or periodically at set intervals and/or on demand, and then the cameras 140 and/or 142 transmit the collected image data to the system computing device 110 and/or to the database 112. The image data may be transmitted periodically, e.g., every second or every minute, at pre-determined time intervals, and/or in any suitable time increment, which enables the BE system 100 to function as described herein.

In the exemplary embodiment, the trackside cameras 140 are positioned about a perimeter of the track 106. More specifically, in the exemplary embodiment, the trackside cameras 140 are substantially vertically aligned with the animals 104. In other embodiments, cameras 140 are oriented at other alignments. In some embodiments, each trackside camera 140 may be able to capture a side view of the animal. For example, the trackside cameras 140 may capture image data at an angle that is generally perpendicular to the side of the animal. In the exemplary embodiment, the trackside cameras 140 are positioned at substantially uniform increments around the perimeter of the track 106. In some embodiments, the trackside cameras 140 are variably positioned at sufficient spacing to ensure that the animals 104 on the track 106 are always in view of at least one of the trackside cameras 140. For example, in some embodiments, the trackside cameras 140 are spaced about forty meters apart. Alternatively, trackside cameras 140 may be positioned at any location relative to the track 106 and at any proximity to animals on the track, *e.g*., within 500 feet of the animal, which enables the trackside cameras 140 to capture images of the animal 102 with a high level of detail at all locations on the track 106. Positioning cameras 140 as such enables key points to be identified, such as anatomical markers 132, on the animal, as described in more detail below. In some embodiments, the camera system 114 includes at least twelve trackside cameras 140. In some embodiments, the trackside cameras 140 may collect images at a rate of 15 frames/second - 120 frames/second.

Each of the plurality of overhead cameras 142 may include an array of six independently controlled cameras 144. In some embodiments, the overhead cameras 142 may move, *e.g.,* flex and extend, rotate, or translate, to enable the view of the overhead camera 142 or a view of any of the six individual independently controlled cameras 144 to be variably changed. The overhead cameras 142 are positioned vertically oriented, e.g., aligned upwardly, relative to the vertical position of the trackside cameras 140. The overhead cameras 142 may be used to generate an artificial pan tilt zoom (PTZ) video of each of the animals 104. In the exemplary embodiment, the overhead cameras 142 are oriented to collect images of a top of the animal 102, *e.g.,* a back of the animal 102, and/or a side perspective view of the animal 102. In some embodiments, the overhead cameras 142 are positioned at the ends 146 of the track 106 and/or on either side 148 of the track 106. The camera system 114 includes enough overhead cameras 142 to enable the cameras 142 to capture the entirety, or a substantial amount, of the track 106. The overhead cameras 142 may be positioned to enable any area of the track 106 that may contain animals 104 to be captured. In some embodiments, the camera system 114 includes at least four overhead cameras 142. Alternatively, camera system 114 may include any number of overhead cameras 142 that enables system 100 to function as described herein. The overhead cameras 142 may collect images at a rate of 15 frames/second - 120 frames/second. The cameras 140 and/or 142 may collect images at different rates or at the same capture rate. In some embodiments, the overhead cameras 142 may collect image data used to determine a position, a speed, and/or stride data, of each animal 102 being monitored, as described in detail below.

In some embodiments, the system computing device 110 may build a training dataset that the system computing device 110 may use to generate the BE model 116, *e.g.,* train and/or tune. The BE model 116 includes one or more model inputs and one or more model outputs. The training dataset may include one or more of the following i) a plurality of historical image data, ii) historical anatomical markers 132 identified using the historical image data, iii) historical determined biomechanical metrics, and/or iv) historic biomechanical assessments or historical outcomes, as described below. The system computing device 110 may build the training dataset by retrieving a reduced size dataset from a complete historical dataset, *e.g.,* such as all the camera data collected over a period of time. The reduced data size of the training data set improves computational efficiency and reduces computation time associated with training the BE model 116 using the training dataset. The system computing device 110 may reduce the data size of the training data set by filtering the complete historical dataset according to one or more criteria, e.g., image quality, animals contained in the images, etc.

In some embodiments, the BE model may be a single model. In some embodiments, the BE model may include a plurality of model used in combination and/or in isolation.

Model inputs may include one or more of the following, i) subject image data, collected by the camera system 114, ii) identified anatomical markers 132 located on the subject image data, iii) determined biomechanic metrics, and/or iv) one or more risk factor metrics, described below. Model inputs may be applied to the BE model 116 in order to evaluate the biomechanical performance of a subject animal 102 over an elapsed period of time. Subject image data may be collected over the elapsed time period during the evaluation of each animal 102 being monitored. Model outputs may include biomechanical assessment metrics. The output biomechanical assessment metrics may include one or more of a risk score, an asymmetry score, and a foreleg lameness score, indicating a level of injury or pathology. In some embodiments, the risk score may be an aggregate of other determined scores. The Foreleg Lameness Score may be an aggregate score that represents the likelihood of a foreleg injury based on the vertical displacement, lateral movement, or rate of change of the vertical displacement etc., of the movement of the horse's head. The Asymmetry Score may be an aggregate score that represents the symmetry of the horse's overall movement. For example, a lack of symmetry in the horse's overall gait is indictive of an animal injury. Alternatively, or in addition, another output biomechanical assessment metrics may be monitored enabling an evaluation of an animal 102 by BE model 116.

In some embodiments, the system computing device 110 may receive or determine one or more risk factor metrics. Risk factor metrics may be indicative of a pathology or injury of an animal or a group of animals. Risk factor metrics may be associated with a group or a plurality of animals, indicating the interindividual variability across the plurality of animals. In some embodiment, risk factor metrics may include an average and/or a standard deviation associated with a determine metric or a measured value across multiple animal. In a first example, a risk factor metric may include a threshold displacement, in the vertical direction, of the head of the animal 102. For example, if the vertical displacement of the head of the animal 102 exceeds a predefined threshold displacement, then the animal 102 may be at a risk of

In embodiments described herein, the system computing device 110 may build a training dataset that will be used to train and/or tune the BE model 116. The training dataset may include historic image data, collected over a period of time, of one or more animals 104, or alternatively of a single specific animal. The system computing device 110 may build the training dataset by storing relative positions of historic anatomical marker 132 for the historic images, historic determined biomechanic metrics, pre-defined risk factors, and/or historic determined assessment metrics. In some embodiments, the system computing device 110 may build the training dataset by including historic pathology or injury diagnosis. In some embodiments, the BE model may be enabled to predict race placements or results, e.g., based on average speed, other determined metrics, or biomechanic assessment metric.

In some embodiments, the BE model 116 may be a machine learning model that includes one or more neural networks, such as a convolutional neural network, a deep learning neural network, or the like. The neural network may have one or more layers of nodes, and the model parameters adjusted during training may be respective weight values applied to one or more inputs to each node to produce a node output. In other words, the nodes in each layer may receive one or more inputs and apply a weight to each input to generate a node output. The node inputs to the first layer may correspond to the model input data fields, and the node outputs of the final layer may correspond to the at least one output of the model, intended to predict the at least one result data field. One or more intermediate layers of nodes may be connected between the nodes of the first layer and the nodes of the final layer.

As the BE model is trained using the training dataset, a suitable backpropagation algorithm may adjust the weighting assigned in each node layer to facilitate reducing any error between the at least one output and the corresponding result data field. In this fashion, the machine learning model is trained to produce outputs that reliably predict the corresponding result data field. Alternatively, the machine learning model may have any other suitable structure. In some embodiments, the BE model may be trained by automatically discovering and properly weighting complex, second or third order, and/or otherwise nonlinear interconnections between the model input data fields and the at least one output. Absent the machine learning model, such connections are unexpected and/or undiscoverable by human analysts.

In some embodiments, the BE system 100 may create one or more different BE models 116. In some embodiments, the system computing device 110 may generate an animal specific BE model, trained historic image data collected over a period time for the specific animal 102. The training data for the animal specific BE model may have reduced data size compared to all the collected training data, reducing computational times. The specific BE models may be used to evaluate changes in the biomechanical performance of the particular animal 102. For example, the specific BE model output may include an indication that the top speed of the animal 102 has changed, that the normal gait pattern of the animal 102 has changed, and the like. In some embodiments, the specific BE model may also generate outputs that are similar to, or are the same as, the BE model 116. The animal specific BE model may be used to generate an animal profile representative of the animal's normal gait, normal behavior, normal movement patterns, etc., creating a baseline for which to detect changes in the animal biomechanical performance or health.

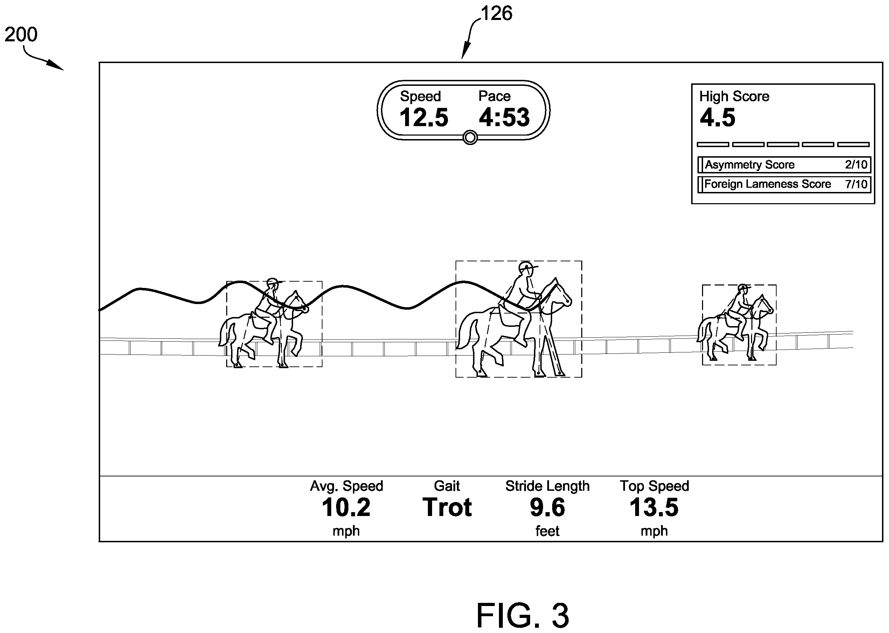

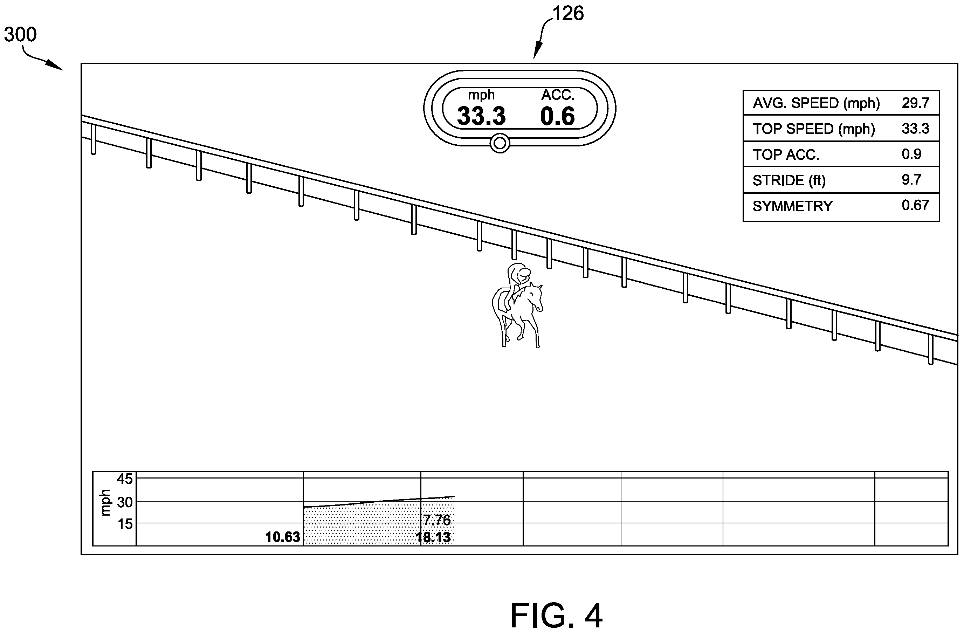

The system computing device 110 may generate one or more notification messages that the system computing device 110 may transmit to the other computing devices. The notification message may include one or more of the model outputs. The system computing device 110 may also add one or more of the model inputs to the notification message. In some embodiments, the notification message may include image data, video data, identified anatomical marker 132, *e.g*., position, or anatomical markers 132 overlaid on the image data, determined metrics (*e.g*., velocity, acceleration, etc.), and/or assessment metrics (*e.g.,* a pathology or injury warning). In some embodiments, The notification messages may also include a recommendation, *e.g*., rest the animal, seek intervention or medical evaluation by veterinarian 120. The notification message may include instructions that cause the user interface to display one or more views of the GUI 126, *see* FIGS. 3 and 4. In some embodiments, the GUI 126 may display the information contained in the notification message.

With reference to FIGS. 3 and 4, in the exemplary embodiments, the BE system 100 includes one or more GUIs 126 that may be displayed on a computing device, *e.g.,* the system computing device 110 and/or the veterinarian computing device 122, for example, for displaying information, such as determined metrics and/or assessment metrics. In some embodiments, the GUI 126 may display images and/or video and in some embodiments, the anatomical markers 132 may be overlaid across the images and/or video. The GUI 126 may include one or more different views, such as a first view shown in FIG. 3 and a second view shown in FIG. 4.

In reference to FIG. 3, an exemplary view 200 of the GUI 126 is illustrated. The view 200 displays image data collected by the camera system 114. The GUI 126 may display isolated image frames and/or a plurality of image frames, such as via a video stream. The GUI 126 may also overlay, onto the displayed image, one or more anatomical markers 132. In some embodiments, the anatomical markers 132 may be color-coded to help in identifying a specific animal 102 captured in the image. In some embodiments, the displayed image data may include more than one animal, and as such, each identified animal 102 may have a different color for use as an anatomical marker 132. The exemplary view 200 displayed may include a risk score, indicative of a level of injury or pathology. In some embodiments, the risk score may be an aggregate of other determined scores. The Foreleg Lameness Score may be an aggregate score that represents the likelihood of a foreleg injury based on the shape of the movement of the horse's head. The Asymmetry Score may be an aggregate score that represents the symmetry of the horse's overall movement. For example, a lack of symmetry in the horse's overall gait is indictive of an animal injury. In some embodiments, the magnitude of score may indicate a level of severity of the identified pathology or injury, e.g., a higher score indicates a higher severity level. The exemplary view 200 may include additional information such as the name of animal, a date of image collection, and/or a location of the image collection, for example. The view 200 may also include the display speed, in miles per hour, and/or the pace in minutes/mile, for example. The view 200 may also display an overhead schematic of the track 106 and a representative marker showing the position of the animal 102 relative to the overhead schematic. The view 200 may display determined biomechanic metrics, such as, for example, an average speed, a type of gait, a stride length, and/or a top speed, for example.

In reference to FIG. 4, an exemplary view 300 of the GUI 126 is illustrated. View 300 may display image data, collected by camera system 114. The view 300 may display a timeline, *e.g.,* a timeline of a race, wherein the timeline illustrates the speed of the animal 102 over the timeline of the race.

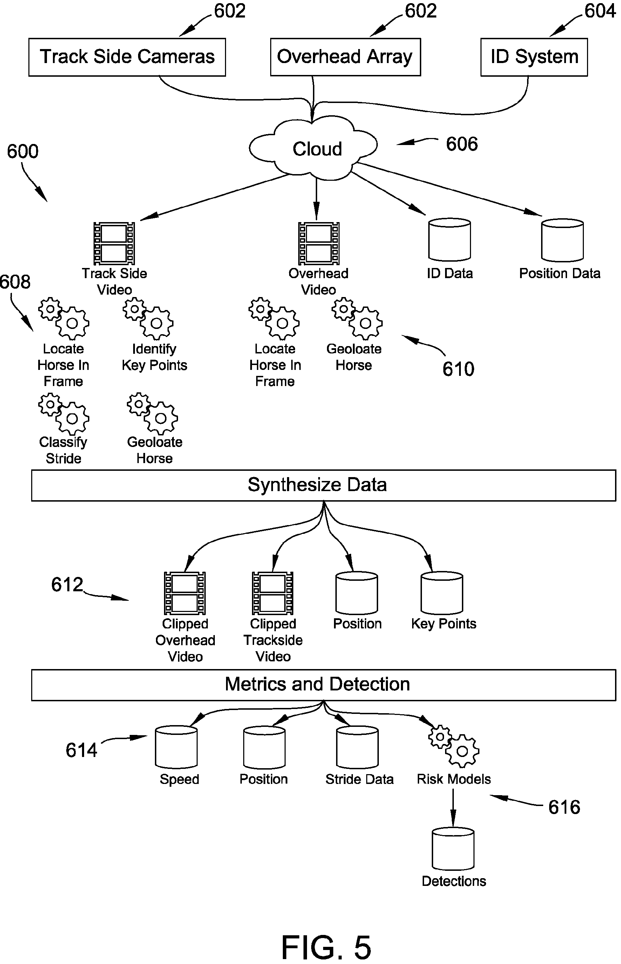

FIG. 5 is a schematic flowchart of an exemplary process 600 that may be used with the BE system 100 (shown in FIG. 1). Any portion of the process 600 may be performed by the system computing device 110. The process 600 includes collecting 602 image data using the trackside cameras 140 and the overhead cameras 142. Process 600 may also include using the identification system 130 to identify 604 and/or to locate a specific animal 102 within the image data, *e.g*., within individual image frames. Process 600 may also include storing 606 the collected image data to the database 112 and/or storing image data with one or more identified animals 104, identified by the identification system 130.

In some embodiments, process 600 includes analyzing 608 image data collected by the trackside camera. Analyzing 608 may include locating and/or identifying, using the image data collected by the trackside cameras 140, a specific animal 102 in one or more image frames collected by the trackside camera and then identifying anatomical markers 132 on the identified animals 104 in the image frames collected by the trackside camera. Analyzing 608 may further include using image data collected by the trackside cameras 140 to classify a type of gait and to geolocate the identified animal 102 relative to a global coordinate system positioned on the track 106.

Process 600 includes analyzing 610 the image data collected by the overhead cameras 142. Analyzing 610 may include using the image data collected by the overhead cameras 142 to locate and/or identify a specific animal 102 in one or more image frames collected by the overhead camera, and then geolocate the position of the animal, e.g., in reference to a global coordinate system on the track 106. In some embodiments, process 600 includes using either, or both, of the image data collected by the trackside cameras 140 and the overhead cameras 142 to classify stride length, identify anatomical markers 132, and/or geolocate the animal 102 on the track 106.

Process 600 further include synthesizing 612 or compiling the image data collected by the overhead cameras 142, image data collected by the trackside cameras 140, the geolocated position data determined above, and the identified anatomical markers 132. Process 600 also includes using the synthesized data to determine 614 biomechanic metrics, *e.g*., speed, etc.

Process 600 includes applying 616 model inputs to the BE model 116, to cause the BE model 116 to output one or more biomechanical assessment metrics.

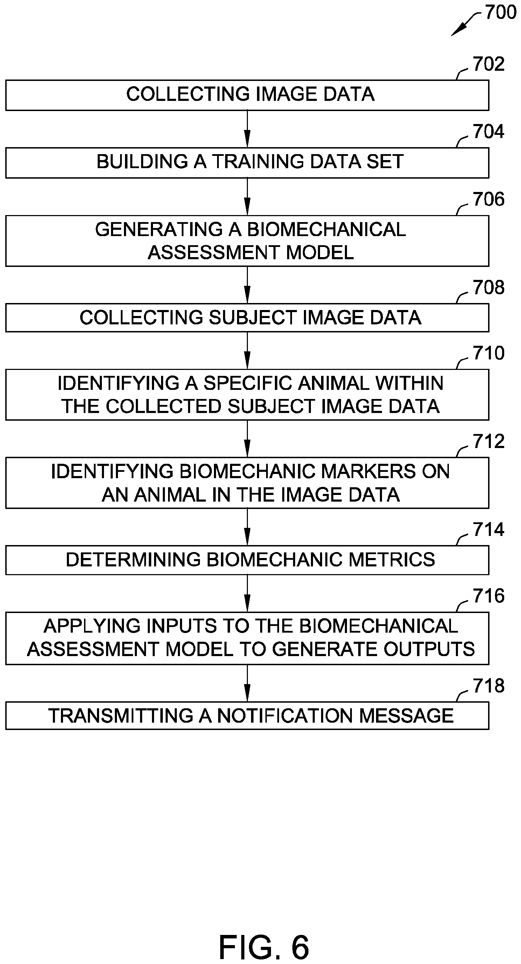

FIG. 6 is process flow diagram of an exemplary biomechanic assessment method 700 for assessing the biomechanics of an animal 102 for use with the BE system 100 (shown in Fig. 1). Method 700 may be implemented, at least in part, by a computing device, *e.g.,* the system computing device 110 and/or the veterinarian computing device 122, for example.

In some embodiments, the method 700 includes collecting 702 image data of at least one animal 102 as the animal 102 moves, *e.g*., trains, exercises, or races, within the track 106. Collecting 702 images may include using the cameras, *e.g*., the trackside camera and/or the overhead camera, to capture images, and then transmitting the collected images to the system computing device 110. In some embodiments, the method 700 includes collecting 702 image data over a pre-defined period of time, *e.g*., weeks, months, etc. In some embodiments, the method 700 includes collecting 702 image data each time an animal 102 is moving within the track 106, *e.g.,* each time an animal 102 is training, competing, or exercising. In some embodiments, the method 700 includes the system computing device 110 storing the image data to the database 112, to create a historical database 112, for example. In some embodiments, the method includes the cameras transmitting, directly, the image data to the database 112.

In the exemplary embodiment, the method 700 includes building a training dataset. The training dataset may subsequently be used to generate a biomechanic evaluation model, described in more detail below. Building 704 the training dataset may include the system computing device 110 compiling the collected historic image data. Building 704 the training data set may also include the system computing device 110 identifying anatomical markers 132 on the historic image data, and then using a system computing device 110 to add the identified anatomical markers 132 to the training data set. In some embodiments, the method 700 further includes identifying an animal 102 in the historic image data, and then using the system computing device 110 to add the animal identifier to the training data set. The method 700 may also include determining, using the system computing device 110, one or more historic biomechanic metrics, such as, but not limited to, the velocity, the acceleration, the type of gait, etc., based on the historic image data. The system computing device 110 then adds the determined historic biomechanic metrics to the training dataset, such that the training dataset is updated continuously. In some embodiments, method 700 may further include the system computing device 110 determining, *e.g*., receiving and/or calculating, one or more risk factor metrics, and then adding the determined risk factor metrics, using the system computing device 110, to the training dataset. In some embodiments, the method 700 includes receiving one or more risk factor metrics from the veterinarian computing device 122. In other embodiments, the risk factor metrics may be received/retrieved from other sources. Examples of risk factor metrics and determined historic biomechanic metrics are described above. Building the training data set may further include the system computing device 110 continuously adding historic assessment metrics and/or historic outcomes to the training data set. Accordingly, the training data set may include one or more historic model inputs and one or more historic model outputs. Building 704 the training dataset may include the system computing device 110 retrieving a reduced size dataset from a complete historical dataset. The historical dataset may include all image data collected over a period of time.

In the exemplary embodiment, method 700 further includes generating 706, using the system computing device 110, the BE model 116. Generating 706 the BE model 116 may include training the BE model 116 using the built training dataset. Generating 706 the BE model 116 may also include any process, such as training or tuning, to generate the BE model 116 with an error reduced below an acceptable or predefined threshold. Generating 706 the BE model 116 may include adjusting, *e.g*., increasing or decreasing, one or more weighting factors in order to adjust the influence of various training model inputs. Building the BE model 116 may include performing, using the system computing device 110, one or more other suitable processes to tune and/or train the BE model 116.

In the exemplary embodiment, method 700 further includes collecting 708 subject image data, associated with a particular animal. Subject image data may refer to data collected during a particular period of time, *e.g*., during a training session or during a race, etc., or during any period of time that a user wishes to evaluate the biomechanical performance of an animal. In some embodiments, the subject image data may be collected over longer periods of time, such as weeks or days, leading up to a race event. In some embodiments, the system computing device 110 may subsequently add the subject image data to the historical image data and/or to the training dataset.

In the exemplary embodiment, method 700 further includes determining, using the system computing device 110, one or more model inputs to be applied to the BE model 116. Determining model inputs may include identifying 710 a specific animal 102 within the collected subject image data. Determining model inputs may further include identifying 712 anatomical markers 132 on the subject image data. In some embodiments, determining one or more model inputs includes the system computing device 110 determining 714 one or more biomechanic metrics, *e.g*., based on the subject image data and/or the identified anatomical markers 132. As described above, determined biomechanic metrics may include the type of gait, the speed, and/or the acceleration, for example. The BE model 116 inputs may include one or more of the following: i) subject image data with or without an identified animal 102 contained within the image data, ii) identified anatomical markers 132, and/or iii) determined biomechanic metrics, for example. Method 700 may include determining any other suitable inputs to be applied to the BE model 116.

In the exemplary embodiment, method 700 include applying 716, using the system computing device 110, the one or more determined model inputs to the BE model 116. Applying 716 the determined inputs to the BE model 116 includes the BE model 116 generating one or more model outputs. Model outputs may include, for example, one or more biometric assessment metrics, such as a warning, a proposed diagnosis, a recommendation, and/or other biometric assessment metrics as described above.

In the exemplary embodiments, method 700 may include generating, using the system computing device 110, one or more notification message including one or more of the BE model 116 outputs, *e.g.,* the biometric assessment metrics, and/or the determined metrics. In some embodiments, the messages may include subject image data.

In the exemplary embodiment, method 700 may further include transmitting 718, using the system computing device 110, the notification messages to one or more computing devices, such as, for example, the veterinarian computing device 122. The messages may be viewed and/or analyzed by an interested user, *e.g.,* a veterinarian 120, to make further recommendation, e.g., regarding treatment of the animal.

In some embodiments, the notification messages may include instructions that cause a computing device to display one or more of the GUI 126 for displaying model output, subject image data and/or video, and/or determined metrics in a manner that is intuitive and/or easily interpreted by a user. Examples of the GUI 126 are described above.

In some embodiments, method 700 may further include the system computing device 110 periodically updating the historic image data, updating the training dataset, and/or periodically retraining the model using the updated training data set.

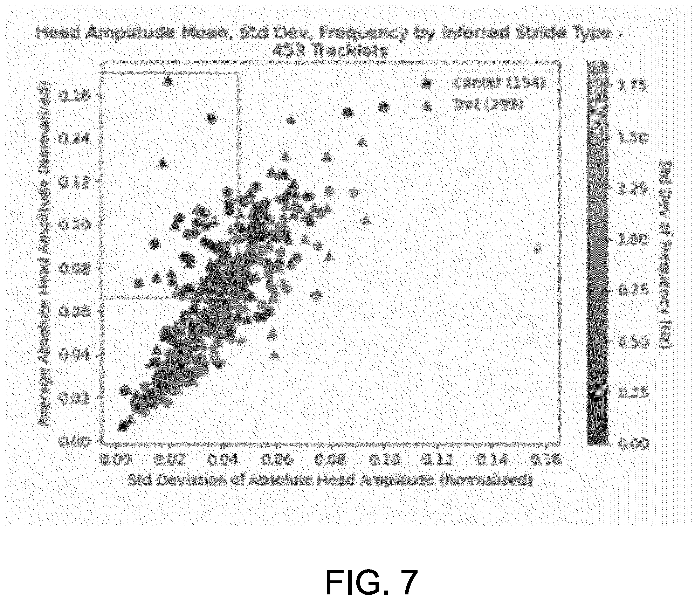

FIG. 7 is a graph illustrating exemplary average absolute head amplitude (normalized) versus standard deviation of absolute head amplitude (normalized) and standard deviation of frequency. The averages may be used as model inputs or model training data. The averages may be used to determine a biomechanic risk factor.

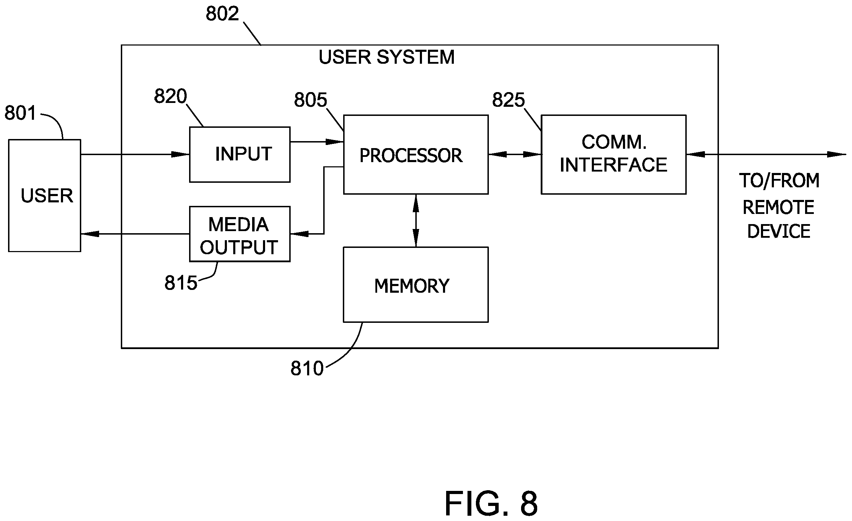

FIG. 8 illustrates an exemplary configuration of a user computing device, such as veterinarian computing device 122 (shown in FIG. 1), that is configured to transmit and/or receive data from the system computing device 110 (shown in FIG. 1). In the exemplary embodiment, user system 802 includes a processor 805 for executing instructions. In some embodiments, executable instructions are stored in a memory 810. Processor 805 may include one or more processing units, for example, a multi-core configuration. Memory 810 is any device that enables information such as executable instructions and/or written works to be stored and retrieved. Memory 810 may include one or more computer readable media.

User system 802 also includes at least one media output component 815 for presenting information to user 801. User 801 may include but is not limited to only including the veterinarian 120. Media output component 815 is any component capable of conveying information to user 801. For example, media output component 815 may be a display component configured to display data associated with the model outputs, images, or video, and/or data contained in the notification message, described above in reference to FIGS. 1-4, or any other suitable data in the form of reports, dashboards, communications, and the like. In some embodiments, media output component 815 includes an output adapter such as a video adapter and/or an audio adapter. An output adapter is operatively coupled to processor 805 and operatively connectable to an output device, such as a display device, a liquid crystal display (LCD), organic light emitting diode (OLED) display, or "electronic ink" display, or an audio output device, a speaker, or headphones.

In some embodiments, user system 802 includes an input device 820 for receiving input from user 801. Input device 820 may include, for example, a keyboard, a pointing device, a mouse, a stylus, a touch sensitive panel, a touch pad, a touch screen, a gyroscope, an accelerometer, a position detector, an audio input device, a fingerprint reader/scanner, a palm print reader/scanner, an iris reader/scanner, a retina reader/scanner, a profile scanner, or the like. A single component, such as a touch screen, may function as both an output device of media output component 815 and input device 820. User system 802 may also include a communication interface 825, which is communicatively connectable to a remote device such as server computing device 110 (shown in FIG. 1). Communication interface 825 may include, for example, a wired or wireless network adapter or a wireless data transceiver for use with a mobile phone network, Global System for Mobile communications (GSM), 3G, or other mobile data network or Worldwide Interoperability for Microwave Access (WIMAX).

Stored in memory 810 are, for example, computer readable instructions for providing a user interface to user 801 via media output component 815 and, optionally, receiving and processing input from input device 820. A user interface may include, among other possibilities, a web browser, and client application. Web browsers enable users, such as user 801, to display and interact with media and other information typically embedded on a web page or a website from the veterinarian computing device 122. A client application enables user 801 to interact with the BE system 100.

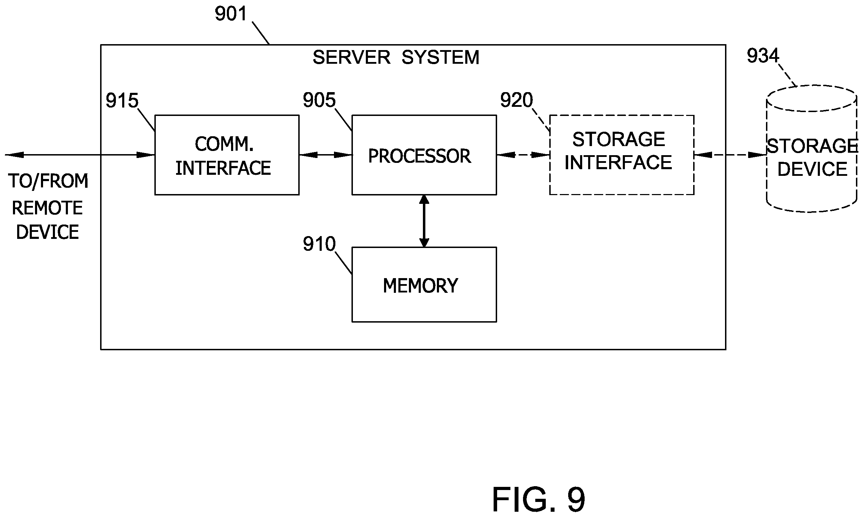

FIG. 9 illustrates an exemplary configuration of a server system 901 such as system computing device 110 (shown in FIG. 1). In some embodiments, the server system 901 may include the veterinarian computing device 122. Server system 901 includes a processor 905 for executing instructions. Instructions may be stored in a memory 910, for example. Processor 905 may include one or more processing units (*e.g*., in a multi-core configuration) for executing instructions. The instructions may be executed within a variety of different operating systems on the server system 901, such as UNIX, LINUX, Microsoft Windows^{®}, etc. More specifically, the instructions may cause various data manipulations on data stored in storage device 934 (*e.g*., create, read, update, and delete procedures). It should also be appreciated that upon initiation of a computer-based method, various instructions may be executed during initialization. Some operations may be required in order to perform one or more processes described herein, while other operations may be more general and/or specific to a particular programming language (*e.g*., C, C#, C++, Java, or other suitable programming languages, etc.).

Processor 905 is operatively coupled to a communication interface 915 such that server system 901 is capable of communicating with a remote device, such as a user system 800 or another server system 901. For example, communication interface 915 may receive communications from other computing devices of the BE system 100 via a plurality of network connections, as illustrated in FIG. 1.

Processor 905 may also be operatively coupled to a storage device 934. Storage device 934 is any computer-operated hardware suitable for storing and/or retrieving data. In some embodiments, storage device 934 is integrated in server system 901. In other embodiments, storage device 934 is external to server system 901 and is similar to database 112 (shown in FIG. 1). For example, server system 901 may include one or more hard disk drives as storage device 934. In other embodiments, storage device 934 is external to server system 901 and may be accessed by a plurality of server systems 901. For example, storage device 934 may include multiple storage units such as hard disks or solid-state disks in a redundant array of inexpensive disks (RAID) configuration. Storage device 934 may include a storage area network (SAN) and/or a network attached storage (NAS) system.

In some embodiments, processor 905 is operatively coupled to storage device 934 via a storage interface 920. Storage interface 920 is any component capable of providing processor 905 with access to storage device 934. Storage interface 920 may include, for example, an Advanced Technology Attachment (ATA) adapter, a Serial ATA (SATA) adapter, a Small Computer System Interface (SCSI) adapter, a RAID controller, a SAN adapter, a network adapter, and/or any component providing processor 905 with access to storage device 934.

Memory 910 may include, but is not limited to, random access memory (RAM) such as dynamic RAM (DRAM) or static RAM (SRAM), read-only memory (ROM), erasable programmable read-only memory (EPROM), electrically erasable programmable read-only memory (EEPROM), and non-volatile RAM (NVRAM). The above memory types are exemplary only and are thus not limiting as to the types of memory usable for storage of a computer program.

In the exemplary embodiments, the BE model enables the use of historic image data, collected over a period of time, *e.g*., weeks or months, in order to assess the biomechanical performance of an animal. The trained BE model is enabled to determine changes in the normal animal, (*e.g*., normal interindividual behavior averaged across a plurality of animals and/or normal intraindividual behavior for a single animal) specific movements, gait patterns, or normal (*e.g*., average) speed for a gait type, as well as identify pathologies and/or injuries. The BE model is trained and tuned, iteratively, using a training dataset including historical image data and historical outcomes, *e.g*., historical pathologies and/or injuries. In the exemplary embodiment, the system includes a camera system having two different sets of cameras each set having a different field of view, enabling the system to identify and track a specific animal no matter the location of the animal on the track and even if there is a plurality of animals on the track. The system includes an identification system that is enabled to identify a specific animal, even in the event that an image frame captures a plurality of animals.

The above description is meant to be exemplary only, and one skilled in the art will recognize that changes may be made to the embodiments described without departing from the scope of the invention disclosed. Modifications, which fall within the scope of the present invention, will be apparent to those skilled in the art, in light of a review of this disclosure, and such modifications are intended to fall within the appended claims.

Although specific features of various embodiments of the disclosure may be shown in some drawings and not in others, this is for convenience only. In accordance with the principles of the disclosure, any feature of a drawing may be referenced and/or claimed in combination with any feature of any other drawing.

This written description uses examples to disclose the embodiments of systems and methods, including the best mode, and also to enable any person skilled in the art to practice the systems and methods, including making and using any devices or systems and performing any incorporated methods. The patentable scope of the systems and methods is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Further aspects of the invention are provided by the subject matter of the following clauses:
1. A computer system for assessing the biomechanical performance of an animal, said computer system comprising at least one processor in communication with at least one memory device, wherein said processor is programmed to:
   receive current image data from at least one camera of at least one animal;
   identify with specificity an animal within the received image data;
   identify at least one biomechanic marker on the identified animal;
   track the position of each identified biomechanic marker;
   determine a biomechanic metric based on each identified biomechanic marker;
   apply, to a biomechanic evaluation model, i) the tracked position of each biomechanic marker, and ii) the determined biomechanic metric, to generate an output including at least one biomechanic assessment metric for use in assessing the performance of the identified animal; and
   transmit one or more notification messages to at least one computing device, wherein each notification message includes at least one generated output biomechanic assessment metric.
2. The computer system of any preceding clause, wherein the processor is further configured to:
   retrieve historic image data;
   identify at least one animal within the retrieved historic image data;
   identify at least one historic biomechanic marker on each animal identified in the retrieved historic image data;
      determine at least one historic biomechanic metric based on the tracked position of each biomechanic marker; and
   generate a training dataset including at least one of i) the historic tracked position, ii) the determined historic biomechanic metric, and iii) one or more biomechanic risk factors.
3. The computer system of any preceding clause, wherein the processor is further configured:
   train, using the generated training dataset, the biomechanic evaluation model for receiving an input including at least one of i) a current biomechanic marker position and ii) a determined biomechanic metric, and output one or more of the biometric assessment metrics.
4. The computer system of Claim 1, wherein determining the biomechanic metric includes determining at least one of a velocity of a biomechanic marker, an acceleration of a biomechanic marker, a type of gait, a stride length, an average speed, a top speed, a center of mass of an anatomical segment, a velocity of an anatomical segment, an acceleration of an anatomical segment, a relative position of two or more biomechanic markers and a vertical displacement of at least one biomechanic marker, associated with the animal being assessed.
5. The computer system of any preceding clause, wherein identifying the at least one biomechanic marker on the identified animal includes at least one of identifying a joint anatomical marker and identifying a body segment marker.
6. The computer system of any preceding clause, wherein receiving image data includes receiving at least first image data from at least one first camera oriented at a first perspective relative to the animal and receiving at least second image data from at least one second camera oriented at a second perspective that is vertically displaced from the first perspective.
7. The computer system of any preceding clause, wherein generating the output including at least one biomechanic assessment metric includes at least one of a risk score, a lameness score, and an asymmetry score.
8. The computer system of any preceding clause, wherein generating the training dataset including one or more biomechanic risk factors includes at least one threshold vertical displacement of an anatomical marker located in proximity to the head of the animal.
9. The computer system of any preceding clause, wherein assessing the biomechanical performance of an animal includes assessing the biomechanical performance of a horse.
10. The computer system of any preceding clause, wherein transmitting one or more notification messages to one or more computing devices includes transmitting one or more notification messages to a computing device associated with a veterinarian.
11. The computer system of any preceding clause, wherein transmitting the one or more notification messages includes transmitting one or more determined biomechanic metrics.
12. The computer system of any preceding clause, wherein the processor is further programmed to:
   display on a computer device one or more images of an identified animal overlayed with one or more identified biomechanic markers.
13. A method for assessing the biomechanics of an animal, the method implemented using a computer device including a processor in communication with a memory device, the method includes:
   receiving current image data from at least one camera of at least one animal;
   identifying with specificity an animal within the received image data;
   identifying at least one biomechanic marker on the identified animal;
   tracking the position of each identified biomechanic marker;
   determining a biomechanic metric based on each identified biomechanic marker;
   applying, to a biomechanic evaluation model, i) the tracked position of each biomechanic marker, and ii) the determined biomechanic metric, to generate an output including at least one biomechanic assessment metric for use in assessing the performance of the identified animal; and
   transmitting one or more notification messages to at least one computing device, wherein each notification message includes at least one generated output biomechanic assessment metrics.
14. The method of any preceding clause, wherein the method further includes:
   retrieving historic image data;
   identifying at least one animal within the retrieved historic image data;
   identifying at least one historic biomechanic marker on each animal identified in the retrieved historic image data;
   determining at least one historic biomechanic metric based on the tracked position of each biomechanic marker; and
   generating a training dataset including at least one of i) the historic tracked position, ii) the determined historic biomechanic metric, and iii) one or more biomechanic risk factors.
15. The method of any preceding clause, wherein the method further comprises:
   training, using the generated training dataset, the biomechanic evaluation model for receiving an input including at least one of i) a current biomechanic
   marker position and ii) a determined biomechanic metric, and output one or more of the biometric assessment metrics.
16. The method of any preceding clause, wherein identifying the at least one biomechanic marker on the identified animal includes at least one of identifying a joint anatomical marker and identifying a body segment marker.
17. The method of any preceding clause, wherein receiving image data includes receiving at least first image data from at least one first camera oriented at a first perspective relative to the animal and receiving at least second image data from at least one second camera oriented at a second perspective that is vertically displaced from the first perspective.
18. The method of any preceding clause, wherein generating the output including at least one biomechanic assessment metric includes at least one of a risk score, a lameness score, and an asymmetry score.
19. A non-transitory computer-readable storage medium including computer-executable instructions embodied thereon for assessing the biomechanics of an animal, wherein when executed by at least one processor, the computer executable instructions cause the at least one processor to:
   receive current image data from at least one camera of at least one animal;
   identify with specificity an animal within the received image data;
   identify at least one biomechanic marker on the identified animal;
   track the position of each identified biomechanic marker;
   determine a biomechanic metric based on each identified biomechanic marker;
   apply, to a biomechanic evaluation model, i) the tracked position of each biomechanic marker, and ii) the determined biomechanic metric, to generate an output including at least one biomechanic assessment metric for use in assessing the performance of the identified animal; and
   transmit one or more notification messages to at least one computing device, wherein each notification message includes at least one generated output biomechanic assessment metric.
20. The non-transitory computer-readable storage medium of any preceding clause, wherein the processor is further configured to:
   retrieve historic image data;
   identify at least one animal within the retrieved historic image data;
   identify at least one historic biomechanic marker on each animal identified in the retrieved historic image data;
   determine at least one historic biomechanic metric based on the tracked position of each biomechanic marker; and
   generate a training dataset including at least one of i) the historic tracked position, ii) the determined historic biomechanic metric, and iii) one or more biomechanic risk factors.

## Claims

1. A computer system for assessing the biomechanical performance of an animal, said computer system comprising at least one processor in communication with at least one memory device, wherein said processor is programmed to:
receive current image data from at least one camera of at least one animal;
identify with specificity an animal within the received image data;
identify at least one biomechanic marker on the identified animal;
track the position of each identified biomechanic marker;
determine a biomechanic metric based on each identified biomechanic marker;
apply, to a biomechanic evaluation model, i) the tracked position of each biomechanic marker, and ii) the determined biomechanic metric, to generate an output including at least one biomechanic assessment metric for use in assessing the performance of the identified animal; and
transmit one or more notification messages to at least one computing device, wherein each notification message includes at least one generated output biomechanic assessment metric.

2. The computer system of Claim 1, wherein determining the biomechanic metric includes determining at least one of a velocity of a biomechanic marker, an acceleration of a biomechanic marker, a type of gait, a stride length, an average speed, a top speed, a center of mass of an anatomical segment, a velocity of an anatomical segment, an acceleration of an anatomical segment, a relative position of two or more biomechanic markers and a vertical displacement of at least one biomechanic marker, associated with the animal being assessed.

3. The computer system of any preceding claim, wherein identifying the at least one biomechanic marker on the identified animal includes at least one of identifying a joint anatomical marker and identifying a body segment marker.

4. The computer system of any preceding claim, wherein receiving image data includes receiving at least first image data from at least one first camera oriented at a first perspective relative to the animal and receiving at least second image data from at least one second camera oriented at a second perspective that is vertically displaced from the first perspective.

5. The computer system of any preceding claim, wherein generating the output including at least one biomechanic assessment metric includes at least one of a risk score, a lameness score, and an asymmetry score.

6. The computer system of any preceding claim, wherein generating the training dataset including one or more biomechanic risk factors includes at least one threshold vertical displacement of an anatomical marker located in proximity to the head of the animal.

7. The computer system of any preceding claim, wherein assessing the biomechanical performance of an animal includes assessing the biomechanical performance of a horse.

8. The computer system of any preceding claim, wherein transmitting one or more notification messages to one or more computing devices includes transmitting one or more notification messages to a computing device associated with a veterinarian.

9. The computer system of any preceding claim, wherein transmitting the one or more notification messages includes transmitting one or more determined biomechanic metrics.

10. A method for assessing the biomechanics of an animal, the method implemented using a computer device including a processor in communication with a memory device, the method includes:
receiving current image data from at least one camera of at least one animal;
identifying with specificity an animal within the received image data;
identifying at least one biomechanic marker on the identified animal;
tracking the position of each identified biomechanic marker;
determining a biomechanic metric based on each identified biomechanic marker;
applying, to a biomechanic evaluation model, i) the tracked position of each biomechanic marker, and ii) the determined biomechanic metric, to generate an output including at least one biomechanic assessment metric for use in assessing the performance of the identified animal; and
transmitting one or more notification messages to at least one computing device, wherein each notification message includes at least one generated output biomechanic assessment metrics.

11. The method of Claim 10, wherein identifying the at least one biomechanic marker on the identified animal includes at least one of identifying a joint anatomical marker and identifying a body segment marker.

12. The method of any one of Claims 10 to 11, wherein receiving image data includes receiving at least first image data from at least one first camera oriented at a first perspective relative to the animal and receiving at least second image data from at least one second camera oriented at a second perspective that is vertically displaced from the first perspective.

13. The method of any one of claims 10 to 12, wherein generating the output including at least one biomechanic assessment metric includes at least one of a risk score, a lameness score, and an asymmetry score.

14. A non-transitory computer-readable storage medium including computer-executable instructions embodied thereon for assessing the biomechanics of an animal, wherein when executed by at least one processor, the computer executable instructions cause the at least one processor to:
receive current image data from at least one camera of at least one animal;
identify with specificity an animal within the received image data;
identify at least one biomechanic marker on the identified animal;
track the position of each identified biomechanic marker;
determine a biomechanic metric based on each identified biomechanic marker;
apply, to a biomechanic evaluation model, i) the tracked position of each biomechanic marker, and ii) the determined biomechanic metric, to generate an output including at least one biomechanic assessment metric for use in assessing the performance of the identified animal; and
transmit one or more notification messages to at least one computing device, wherein each notification message includes at least one generated output biomechanic assessment metric.

15. The non-transitory computer-readable storage medium of Claim 14, wherein the processor is further configured to:
retrieve historic image data;
identify at least one animal within the retrieved historic image data;
identify at least one historic biomechanic marker on each animal identified in the retrieved historic image data;
determine at least one historic biomechanic metric based on the tracked position of each biomechanic marker; and
generate a training dataset including at least one of i) the historic tracked position, ii) the determined historic biomechanic metric, and iii) one or more biomechanic risk factors.
